# EUROPÄISCHE PATENTANMELDUNG

(11) **EP 2 388 595 A2**
(43) Veröffentlichungstag der Anmeldung: **23.11.2011**
(21) Anmeldenummer: 11175127.7
(22) Anmeldetag: 10.08.2007
(51) Int. Cl.: G01N 33/68

(54) **Biomarker für Leberentzündung**

(30) Priorität: 10.08.2006 DE 102006037613; 12.10.2006 DE 102006048249
(62) Teilanmeldung aus: 07801230.9
(71) Anmelder: Schmiegel, Wolff, 44797 Bochum (DE); Sipos, Bence, 24105 Kiel (DE); Sitek, Barbara, 44809 Bochum (DE); Meyer, Helmut E., 45661 Recklinghausen (DE); Stühler, Kai, 50733 Köln (DE)
(72) Erfinder: Schmiegel, Wolff, 44797 Bochum (DE); Sipos, Bence, 24105 Kiel (DE); Sitek, Barbara, 44809 Bochum (DE); Meyer, Helmut E., 45661 Recklinghausen (DE); Stühler, Kai, 50733 Köln (DE); Klöppel, Günter, 24105 Kiel (DE)
(74) Vertreter: Simandi, Claus

(57) **Zusammenfassung**

Die Erfindung betrifft ein Verfahren zur diagnostischen Untersuchung biologischer Proben eines Menschen auf Leberentzündung, insbesondere hepatische Fibrose und / oder Leberzirrhose, wobei die Probe auf eines oder mehrere Proteine als Marker für Leberentzündung, insbesondere hepatische Fibrose und / oder Leberzirrhose, untersucht wird, wobei eine gegenüber dem gesunden Zustand erhöhte oder verringerte Konzentration der Proteine das Vorliegen einer Leberentzündung, insbesondere einer hepatischen Fibrose und / oder Leberzirrhose, anzeigt. Die Proteine sind ausgewählt aus der Gruppe ER60, Vimentin, Actin alpha 1 skeletales Muskelprotein, hMFAP 4, Tropomyosin, PTGES 2, Amyloid P-Komponente, Transgelin, Calponin 1, Homo sapiens p20 Protein, 17 kDa Myosin leichte Kette, H Chain H Igg B12, Prolyl 4-hydroxylase, beta subunit Methylentetrahydrofolatdehydrogenase 1, PRO2619, Aldehyddehydrogenase 1, Fibrinogen alpha-Kette Preproprotein, Fructose-Bisphosphat-Aldolase B, Argininosuccinatsynthetase, Eef1a2, ATP5A1, alpha-2-Actin, Regucalcin, Serumalbumin, mitochondriale Malatdehydrogenase, mitochondrialer Acetoacetyl-CoA-Thiolase oder jeweils eine Teilsequenz davon

SEQUENCE LISTING

<110> Schmiegel, Wolff
      Stühler, Kai
      Meyer, Helmut
      Sipos, Bence
      Sitek, Barbara

<120> Biomarker für Leberentzündung

<130> MEY32-05WOEP-02

<140> 10 2006 037 613.7
<141> 2006-08-10

<160> 7

<170> PatentIn version 3.3

<210> 1
<211> 284
<212> PRT
<213> Homo sapiens

<400> 1

Met Asp Ala Ile Lys Lys Lys Met Gln Met Leu Lys Leu Asp Lys Glu
1               5                   10                  15

Asn Ala Leu Asp Arg Ala Glu Gln Ala Glu Ala Asp Lys Lys Ala Ala
            20                  25                  30

Glu Asp Arg Ser Lys Gln Leu Glu Glu Asp Ile Ala Ala Lys Glu Lys
        35                  40                  45

Leu Leu Arg Val Ser Glu Asp Glu Arg Asp Arg Val Leu Glu Glu Leu
    50                  55                  60

His Lys Ala Glu Asp Ser Leu Leu Ala Ala Glu Glu Ala Ala Ala Lys
65                  70                  75                  80

Ala Glu Ala Asp Val Ala Ser Leu Asn Arg Arg Ile Gln Leu Val Glu
                85                  90                  95

Glu Glu Leu Asp Arg Ala Gln Glu Arg Leu Ala Thr Ala Leu Gln Lys
            100                 105                 110

Leu Glu Glu Ala Glu Lys Ala Ala Asp Glu Ser Glu Arg Gly Met Lys
        115                 120                 125

Val Ile Glu Ser Arg Ala Gln Lys Asp Glu Glu Lys Met Glu Ile Gln
    130                 135                 140

Glu Ile Gln Leu Lys Glu Ala Lys His Ile Ala Glu Asp Ala Asp Arg

145                 150                 155                 160

Lys Tyr Glu Glu Val Ala Arg Lys Leu Val Ile Ile Glu Ser Asp Leu
                165                 170                 175

Glu Arg Ala Glu Glu Arg Ala Glu Leu Ser Glu Gly Lys Cys Ala Glu
            180                 185                 190

Leu Glu Glu Glu Leu Lys Thr Val Thr Asn Asp Leu Lys Ser Leu Glu
        195                 200                 205

Ala Gln Ala Glu Lys Tyr Ser Gln Lys Glu Asp Arg Tyr Glu Glu Glu
    210                 215                 220

Ile Lys Val Leu Ser Asp Lys Leu Lys Glu Ala Glu Thr Arg Ala Glu
225                 230                 235                 240

Phe Ala Glu Arg Ser Val Thr Lys Leu Glu Lys Ser Ile Asp Asp Leu
                245                 250                 255

Glu Asp Glu Leu Tyr Ala Gln Lys Leu Lys Tyr Lys Ala Ile Ser Glu
            260                 265                 270

Glu Leu Asp His Ala Leu Asn Asp Met Thr Ser Ile
        275                 280

<210>  2
<211>  284
<212>  PRT
<213>  Homo sapiens

<400>  2

Met Asp Ala Ile Lys Lys Lys Met Gln Met Leu Lys Leu Asp Lys Glu
1               5                   10                  15

Asn Ala Leu Asp Arg Ala Glu Gln Ala Glu Ala Asp Lys Lys Ala Ala
            20                  25                  30

Glu Asp Arg Ser Lys Gln Leu Glu Glu Asp Ile Ala Ala Lys Glu Lys
        35                  40                  45

Leu Leu Arg Val Ser Glu Asp Glu Arg Asp Arg Val Leu Glu Glu Leu
    50                  55                  60

His Lys Ala Glu Asp Ser Leu Leu Ala Ala Glu Glu Ala Ala Ala Lys
65                  70                  75                  80

Ala Glu Ala Asp Val Ala Ser Leu Asn Arg Arg Ile Gln Leu Val Glu
                85                  90                  95

Glu Glu Leu Asp Arg Ala Gln Glu Arg Leu Ala Thr Ala Leu Gln Lys
        100                 105                 110

Leu Glu Glu Ala Glu Lys Ala Ala Asp Glu Ser Glu Arg Gly Met Lys
        115                 120                 125

Val Ile Glu Ser Arg Ala Gln Lys Asp Glu Glu Lys Met Glu Ile Gln
    130                 135                 140

Glu Ile Gln Leu Lys Glu Ala Lys His Ile Ala Glu Asp Ala Asp Arg
145                 150                 155                 160

Lys Tyr Glu Glu Val Ala Arg Lys Leu Val Ile Ile Glu Ser Asp Leu
                165                 170                 175

Glu Arg Ala Glu Glu Arg Ala Glu Leu Ser Glu Gly Lys Cys Ala Glu
            180                 185                 190

Leu Glu Glu Glu Leu Lys Thr Val Thr Asn Asp Leu Lys Ser Leu Glu
        195                 200                 205

Ala Gln Ala Glu Lys Tyr Ser Gln Lys Glu Asp Arg Tyr Glu Glu Glu
    210                 215                 220

Ile Lys Val Leu Ser Asp Lys Leu Lys Glu Ala Glu Thr Arg Ala Glu
225                 230                 235                 240

Phe Ala Glu Arg Ser Val Thr Lys Leu Glu Lys Ser Ile Asp Asp Leu
                245                 250                 255

Glu Asp Glu Leu Tyr Ala Gln Lys Leu Lys Tyr Lys Ala Ile Ser Glu
            260                 265                 270

Glu Leu Asp His Ala Leu Asn Asp Met Thr Ser Ile
        275                 280

<210>  3
<211>  284
<212>  PRT
<213>  Homo sapiens

<400>  3

Met Asp Ala Ile Lys Lys Lys Met Gln Met Leu Lys Leu Asp Lys Glu
1               5                   10                  15

Asn Ala Ile Asp Arg Ala Glu Gln Ala Glu Ala Asp Lys Lys Gln Ala
            20                  25                  30

Glu Asp Arg Cys Lys Gln Leu Glu Glu Glu Gln Gln Ala Leu Gln Lys

35                  40                  45

Lys Leu Lys Gly Thr Glu Asp Glu Val Glu Lys Tyr Ser Glu Ser Val
    50                  55                  60

Lys Glu Ala Gln Glu Lys Leu Glu Gln Ala Glu Lys Lys Ala Thr Asp
65                  70                  75                  80

Ala Glu Ala Asp Val Ala Ser Leu Asn Arg Arg Ile Gln Leu Val Glu
                85                  90                  95

Glu Glu Leu Asp Arg Ala Gln Glu Arg Leu Ala Thr Ala Leu Gln Lys
            100                 105                 110

Leu Glu Glu Ala Glu Lys Ala Ala Asp Glu Ser Glu Arg Gly Met Lys
        115                 120                 125

Val Ile Glu Asn Arg Ala Met Lys Asp Glu Glu Lys Met Glu Leu Gln
    130                 135                 140

Glu Met Gln Leu Lys Glu Ala Lys His Ile Ala Glu Asp Ser Asp Arg
145                 150                 155                 160

Lys Tyr Glu Glu Val Ala Arg Lys Leu Val Ile Leu Glu Gly Glu Leu
                165                 170                 175

Glu Arg Ser Glu Glu Arg Ala Glu Val Ala Glu Ser Arg Ala Arg Gln
            180                 185                 190

Leu Glu Glu Glu Leu Arg Thr Met Asp Gln Ala Leu Lys Ser Leu Met
        195                 200                 205

Ala Ser Glu Glu Glu Tyr Ser Thr Lys Glu Asp Lys Tyr Glu Glu Glu
    210                 215                 220

Ile Lys Leu Leu Glu Glu Lys Leu Lys Glu Ala Glu Thr Arg Ala Glu
225                 230                 235                 240

Phe Ala Glu Arg Ser Val Ala Lys Leu Glu Lys Thr Ile Asp Asp Leu
                245                 250                 255

Glu Glu Thr Leu Ala Ser Ala Lys Glu Glu Asn Val Glu Ile His Gln
            260                 265                 270

Thr Leu Asp Gln Thr Leu Leu Glu Leu Asn Asn Leu
        275                 280

<210>  4

<211>  284
<212>  PRT
<213>  Homo sapiens

<400>  4

Met Asp Ala Ile Lys Lys Lys Met Gln Met Leu Lys Leu Asp Lys Glu
1               5                   10                  15

Asn Ala Leu Asp Arg Ala Glu Gln Ala Glu Ala Asp Lys Lys Ala Ala
            20                  25                  30

Glu Asp Arg Ser Lys Gln Leu Glu Asp Glu Leu Val Ser Leu Gln Lys
        35                  40                  45

Lys Leu Lys Gly Thr Glu Asp Glu Leu Asp Lys Tyr Ser Glu Ala Leu
    50                  55                  60

Lys Asp Ala Gln Glu Lys Leu Glu Leu Ala Glu Lys Lys Ala Thr Asp
65                  70                  75                  80

Ala Glu Ala Asp Val Ala Ser Leu Asn Arg Arg Ile Gln Leu Val Glu
                85                  90                  95

Glu Glu Leu Asp Arg Ala Gln Glu Arg Leu Ala Thr Ala Leu Gln Lys
            100                 105                 110

Leu Glu Glu Ala Glu Lys Ala Ala Asp Glu Ser Glu Arg Gly Met Lys
        115                 120                 125

Val Ile Glu Ser Arg Ala Gln Lys Asp Glu Glu Lys Met Glu Ile Gln
    130                 135                 140

Glu Ile Gln Leu Lys Glu Ala Lys His Ile Ala Glu Asp Ala Asp Arg
145                 150                 155                 160

Lys Tyr Glu Glu Val Ala Arg Lys Leu Val Ile Ile Glu Ser Asp Leu
                165                 170                 175

Glu Arg Ala Glu Glu Arg Ala Glu Leu Ser Glu Gly Gln Val Arg Gln
            180                 185                 190

Leu Glu Glu Gln Leu Arg Ile Met Asp Gln Thr Leu Lys Ala Leu Met
        195                 200                 205

Ala Ala Glu Asp Lys Tyr Ser Gln Lys Glu Asp Arg Tyr Glu Glu Glu
    210                 215                 220

Ile Lys Val Leu Ser Asp Lys Leu Lys Glu Ala Glu Thr Arg Ala Glu
225                 230                 235                 240

Phe Ala Glu Arg Ser Val Thr Lys Leu Glu Lys Ser Ile Asp Asp Leu
                245                 250                 255

Glu Glu Lys Val Ala His Ala Lys Glu Glu Asn Leu Ser Met His Gln
            260                 265                 270

Met Leu Asp Gln Thr Leu Leu Glu Leu Asn Asn Met
        275                 280

<210>  5
<211>  248
<212>  PRT
<213>  Homo sapiens

<400>  5

Met Ala Gly Leu Asn Ser Leu Glu Ala Val Lys Arg Lys Ile Gln Ala
1               5                   10                  15

Leu Gln Gln Gln Ala Asp Glu Ala Glu Asp Arg Ala Gln Gly Leu Gln
            20                  25                  30

Arg Glu Leu Asp Gly Glu Arg Glu Arg Arg Glu Lys Ala Glu Gly Asp
        35                  40                  45

Val Ala Ala Leu Asn Arg Arg Ile Gln Leu Phe Glu Glu Glu Leu Asp
    50                  55                  60

Arg Ala Gln Glu Arg Leu Ala Thr Ala Leu Gln Lys Leu Glu Glu Ala
65                  70                  75                  80

Glu Lys Ala Ala Asp Glu Ser Glu Arg Gly Met Lys Val Ile Glu Asn
                85                  90                  95

Arg Ala Met Lys Asp Glu Glu Lys Met Glu Ile Gln Glu Met Gln Leu
            100                 105                 110

Lys Glu Ala Lys His Ile Ala Glu Glu Ala Asp Arg Lys Tyr Glu Glu
        115                 120                 125

Val Ala Arg Lys Leu Val Ile Leu Glu Gly Glu Leu Glu Arg Ala Glu
    130                 135                 140

Glu Arg Ala Glu Val Ser Glu Leu Lys Cys Gly Asp Leu Glu Glu Glu
145                 150                 155                 160

Leu Lys Asn Val Thr Asn Asn Leu Lys Ser Leu Glu Ala Ala Ser Glu
                165                 170                 175

Lys Tyr Ser Glu Lys Glu Asp Lys Tyr Glu Glu Glu Ile Lys Leu Leu

180                 185                 190

Ser Asp Lys Leu Lys Glu Ala Glu Thr Arg Ala Glu Phe Ala Glu Arg
        195                 200                 205

Thr Val Ala Lys Leu Glu Lys Thr Ile Asp Asp Leu Glu Glu Lys Leu
    210                 215                 220

Ala Gln Ala Lys Glu Glu Asn Val Gly Leu His Gln Thr Leu Asp Gln
225                 230                 235                 240

Thr Leu Asn Glu Leu Asn Cys Ile
                245

<210>  6
<211>  201
<212>  PRT
<213>  Homo sapiens

<400>  6

Met Ala Asn Lys Gly Pro Ser Tyr Gly Met Ser Arg Glu Val Gln Ser
1               5                   10                  15

Lys Ile Glu Lys Lys Tyr Asp Glu Glu Leu Glu Glu Arg Leu Val Glu
            20                  25                  30

Trp Ile Ile Val Gln Cys Gly Pro Asp Val Gly Arg Pro Asp Arg Gly
        35                  40                  45

Arg Leu Gly Phe Gln Val Trp Leu Lys Asn Gly Val Ile Leu Ser Lys
    50                  55                  60

Leu Val Asn Ser Leu Tyr Pro Asp Gly Ser Lys Pro Val Lys Val Pro
65                  70                  75                  80

Glu Asn Pro Pro Ser Met Val Phe Lys Gln Met Glu Gln Val Ala Gln
                85                  90                  95

Phe Leu Lys Ala Ala Glu Asp Tyr Gly Val Ile Lys Thr Asp Met Phe
            100                 105                 110

Gln Thr Val Asp Leu Phe Glu Gly Lys Asp Met Ala Ala Val Gln Arg
        115                 120                 125

Thr Leu Met Ala Leu Gly Ser Leu Ala Val Thr Lys Asn Asp Gly His
    130                 135                 140

Tyr Arg Gly Asp Pro Asn Trp Phe Met Lys Lys Ala Gln Glu His Lys
145                 150                 155                 160

Arg Glu Phe Thr Glu Ser Gln Leu Gln Glu Gly Lys His Val Ile Gly
                165                 170                 175

Leu Gln Met Gly Ser Asn Arg Gly Ala Ser Gln Ala Gly Met Thr Gly
            180                 185                 190

Tyr Gly Arg Pro Arg Gln Ile Ile Ser
        195                 200

<210>  7
<211>  184
<212>  PRT
<213>  Homo sapiens

<400>  7

Met Ala Asn Lys Gly Pro Ser Tyr Gly Met Ser Arg Glu Val Gln Ser
1               5                   10                  15

Lys Ile Glu Lys Lys Tyr Asp Glu Glu Leu Glu Glu Arg Leu Val Glu
            20                  25                  30

Trp Ile Ile Val Gln Arg Gly Pro Asp Val Gly Arg Pro Asp Arg Gly
        35                  40                  45

Arg Leu Gly Phe Gln Val Trp Leu Lys Asn Gly Val Ile Leu Ser Lys
    50                  55                  60

Leu Val Asn Ser Leu Tyr Pro Asp Gly Ser Lys Pro Val Lys Val Pro
65                  70                  75                  80

Glu Asn Pro Pro Ser Met Val Phe Lys Gln Met Glu Gln Val Ala Gln
                85                  90                  95

Phe Leu Lys Ala Ala Glu Asp Tyr Gly Val Ile Lys Thr Asp Met Phe
            100                 105                 110

Gln Thr Val Asp Leu Phe Glu Gly Lys Asp Met Ala Ala Val Gln Arg
        115                 120                 125

Thr Leu Met Ala Leu Gly Ser Leu Ala Val Thr Lys Asn Asp Gly His
    130                 135                 140

Tyr Arg Gly Asp Pro Asn Trp Phe Met Lys Lys Ala Gln Glu His Lys
145                 150                 155                 160

Arg Glu Phe Thr Glu Ser Gln Leu Gln Glu Gly Lys His Val Ile Gly
                165                 170                 175

Leu Gln Met Gly Ser Asn Arg Gly

            180

## Beschreibung

Die Erfindung betrifft ein Verfahren zur diagnostischen Untersuchung biologischer Proben eines Menschen auf Leberentzündung, insbesondere hepatische Fibrose und / oder Leberzirrhose, wobei die Probe auf eines oder mehrere Proteine als Marker für Leberentzündung, insbesondere hepatische Fibrose und / oder Leberzirrhose, untersucht wird, wobei eine gegenüber dem gesunden Zustand erhöhte oder verringerte Konzentration der Proteine das Vorliegen einer Leberentzündung, insbesondere einer hepatischen Fibrose und / oder Leberzirrhose, anzeigt.

Weltweit sind ca. 170 Mio. Menschen chronisch mit dem Hepatitis C-Virus (HCV) infiziert. Der Verlauf der Krankheit variiert dabei zwischen den Patienten erheblich; während ca. 20 % der Patienten innerhalb von 20 Jahren eine Leberzirrhose entwickeln, ist bei anderen Patienten eine derartige Entwicklung selbst nach noch längeren Zeiträumen nicht zu beobachten. Es konnten eine Reihe von Faktoren identifiziert werden, die die Wahrscheinlichkeit einer hepatischen Fibrose und / oder Leberzirrhose erhöhen, u.a. männliches Geschlecht, Alkoholmissbrauch, Co-Infektion mit HIV oder *Schistosoma mansoni*, genetische Prädisposition und erhöhtes Alter bei der Infektion.

Als Vorläuferzellen sind für die Entwicklung einer Leberfibrose und / oder Leberzirrhose vor allem die hepatischen Stelatzellen (HSC) verantwortlich, die in einer normalen Leber im Ruhezustand insbesondere für die Speicherung von Vitamin A verantwortlich sind. In einer fibrotischen Leber hingegen werden sie aktiviert, proliferieren und entwickeln sich zu myofibroblastartigen Zellen. Diese Myofibroblasten produzieren große Mengen Kollagen, regulieren die Produktion von Matrixmetalloproteinasen (MMPs) herab und zeigen eine erhöhte Expression der physiologischen Inhibitoren der MMPs (TIMPs). Mit zunehmender Kollagenakkumulation entwickelt sich die Fibrose der Leber weiter, was schließlich bis zum Organversagen führen kann.

Zur antiviralen Behandlung einer chronischen Hepatitis C werden insbesondere Peg-Interferon Alfa und Ribavirin eingesetzt. Auch wenn auf diese Weise viele Patienten erfolgreich behandelt werden können, bleibt die Therapie bei mindestens 50 % der Patienten, die mit dem HCV Genotyp 1 infiziert sind, der in der westlichen Welt am weitesten verbreitet ist, erfolglos. Ähnliches gilt für Patienten, die mit dem HCV Genotyp 4 infiziert sind, der in Ägypten häufig vorkommt. Darüber hinaus sind die Kosten der dauerhaften antiviralen Behandlung immens und die Behandlung ist mit erheblichen Nebenwirkungen verbunden. Bei Patienten, die sich in einem weit fortgeschrittenen Stadium befinden, führt die antivirale Behandlung wiederum nicht mehr zum gewünschten Erfolg. Es besteht daher Bedarf, eine Fibrose bei Hepatitis-Patienten und damit die Entstehung einer Leberzirrhose besser diagnostizieren zu können, um dem behandelnden Arzt die Möglichkeit zu verschaffen, zu entscheiden, ob eine antivirale Behandlung sinnvoll und Erfolg versprechend ist.

Bereits in der Vergangenheit wurde eine Reihe nicht-invasiver Marker zum Nachweis von Leberfibrose verwendet, darunter der sog. Acti- oder Fibro-Test, Prokollagen III-Peptid (PIIIP), Hyaluronsäure, Matrixmetalloproteinasen (MMPs) und ihre Inhibitoren (TIMPs) (T. Poynard et al., Expert Rev Mol Diagn. 2005, 5 (1): 15 - 21; V. Leroy et al., J Hep 2001, 35 (1):26). All diese Marker zeigen jedoch nur eine limitierte Sensibilität und Spezifität, weshalb weiterhin Bedarf nach geeigneteren Biomarkern besteht.

Ausgehend vom beschriebenen Stand der Technik stellt sich daher die Aufgabe ein verbessertes Verfahren zur Untersuchung biologischer Proben auf Leberentzündung und / oder hepatische Fibrose und / oder Leberzirrhose zur Verfügung zu stellen, bei dem neuartige Marker verwendet werden.

Die Aufgabe wird erfindungsgemäß gelöst durch ein Verfahren zur Untersuchung biologischer Proben eines Menschen auf Leberentzündung, insbesondere hepatische Fibrose und / oder Leberzirrhose, wobei die Probe auf eines oder mehrere Proteine als Marker für eine Leberentzündung, insbesondere hepatische Fibrose und / oder Leberzirrhose, untersucht wird und wobei ein erhöhter Level der Proteine das Vorliegen einer Leberentzündung, insbesondere hepatische Fibrose und / oder Leberzirrhose, anzeigt, wobei die Proteine ausgewählt sind aus einer Gruppe bestehend aus: ER60, Vimentin, Actin alpha 1 skeletales Muskelprotein, hMFAP4, Tropomyosin, PTGES2, Amyloid-P-Komponente, Transgelin, Calponin 1, Homo sapiens p20 protein, 17 kDa Myosin leichte Kette, H Chain H Igg B12, Prolyl 4-hydroxylase, beta subunit.

Des Weiteren betrifft die Erfindung auch ein entsprechendes Verfahren bei dem ein verringerter Level der Proteine das Vorliegen einer Leberentzündung, insbesondere hepatische Fibrose und / oder Leberzirrhose, anzeigt, wobei in diesem Fall die Proteine ausgewählt sind aus einer Gruppe: Methylentetrahydrofolatdehydrogenase 1, PRO2619, Aldehyddehydrogenase 1, Fibrinogen alpha-Kette Preproprotein, Fructose-Bisphosphat-Aldolase B, Argininosuccinatsynthetase, Eef1a2, ATP5A1, alpha-2-Actin, Regucalcin, Serumalbumin, mitochondriale Malatdehydrogenase, mitochondriale Acetoacetyl-CoA-Thiolase.

Sowohl bei den herauf als auch bei herunterregulierenden Proteinen kann die Untersuchung ebenfalls über die Bestimmung von Teilsequenzen der erfindungsgemäßen Biomarker (auch: Markerproteine) erfolgen. Insbesondere sind solche Teilsequenzen bevorzugt die 60 % der Aminosäuresequenz eines erfindungsgemäßen Biomarker, insbesondere 70 % und mehr, 80 % und mehr, insbesondere 90 bis 95 % umfassen.

Im Rahmen dieser Erfindung umfasst der Begriff der Leberentzündung jede Form der Hepatitis, jedoch insbesondere die hepatische Fibrose bis hin zur Leberzirrhose (siehe zu dem Begriffen z.B. einschlägig Pschyrembel, Klinisches Wörterbuch, 260. Auflage 2004, Berlin). Die hepatische Fibrose und Leberzirrhose ist erfindungsgemäß bevorzugt.

Ferner betrifft die Erfindung ebenfalls die Diagnose von Leberentzündungen, insbesondere hepatische Fibrose und / oder Leberzirrhose, wobei eine Bestimmung mindestens eines Proteins ausgewählt aus der Gruppe bestehend aus: ER60, Vimentin, Actin alpha 1 skeletales Muskelprotein, hMFAP 4, Tropomyosin, PTGES 2, Amyloid P-Komponente, Transgelin, Calponin 1, Homo sapiens p20 Protein, 17 kDa Myosin leichte Kette, H Chain H Igg B12, Prolyl 4-hydroxylase, beta subunit Methylentetrahydrofolatdehydrogenase 1, PRO2619, Aldehyddehydrogenase 1, Fibrinogen alpha-Kette Preproprotein, Fructose-Bisphosphat-Aldolase B, Argininosuccinatsynthetase, Eef1a2, ATP5A1, alpha-2-Actin, Regucalcin, Serumalbumin, mitochondriale Malatdehydrogenase, mitochondrialer Acetoacetyl-CoA-Thiolase oder jeweils eine Teilsequenz davon an einem zu untersuchenden Patienten durchgeführt wird.

Des Weiteren ist eine Kombination solcher erfindungsgemäßen Biomarker bzw. Markerproteine zur erfindungsgemäßen Diagnose möglich.

Die genannten Proteine konnten bei einer Proteomanalyse von fibrotischem Gewebe im Vergleich zu nicht-fibrotischem Gewebe als potentielle Biomarker identifiziert werden. Hierzu wurden mit Hepatitis C infizierten Patienten Leber-Biopsieproben entnommen. Die Proben wurden in einem Handhomogenisator mit Lysispuffer homogenisiert und von DNA und sonstigem Zellmaterial befreit, um ein Proteinkonzentrat zu erhalten. Die Proteine wurden mit einem Farbstoff gelabelt und einer 2D-Polyacrylamid-Gelelektrophorese unterworfen mit einer isoelektrischen Fokussierung in der ersten und einer SDS-Gelelektrophorese in der zweiten Dimension. Die Ergebnisse wurden für fibrotische und nicht-fibrotische Zellen mit Hilfe einer dafür geeigneten Software verglichen, um die Spots zu detektieren und zu quantifizieren, die bei der fibrotischen Probe im Vergleich zur nicht-fibrotischen Probe verstärkt oder verringert waren. Als Software kann beispielsweise die ImageQuant^{™}-Software der Firma GE Healthcare in Verbindung mit der DeCyder-Software derselben Firma durchgeführt werden. Dabei wird die Emission der Farbstoffe, mit denen die Proteine gelabelt wurden, gemessen und ausgewertet.

Die weitere Auswertung erfolgte mit Hilfe von LC-ESI-MS (Flüssigchomatographie-Elektrosprayionisations-Massenspektrometrie). Dabei wurden zunächst die Proteine im Gel, in dem die Proben zuvor aufgetrennt wurden, mit Hilfe von Trypsin in einzelne Peptidfragmente zerlegt. Diese wurden mit Hilfe von Reversed-Phase HPLC voneinander separiert und massenspektrometrisch untersucht, um die einzelnen Proteine zu identifizieren. Selbstverständlich können hierbei auch andere geeignete massenspektrometrische Verfahren angewandt werden, beispielsweise MALDI-TOF-MS.

Bei den Untersuchungen konnten folgende Proteine identifiziert werden, die in fibrotischen Zellen gegenüber nicht-fibrotischen Zellen herauf- (fold change positiv) bzw. herunterreguliert (fold change negativ) wurden:

**Heraufregulierte Proteine:**

| **NCBI-Accession** | **Identifiziertes Protein** | **Fold change** |
|---|---|---|
| IPI00025252.1 | ER60 Protein | 26,9 |
| IPI00418471.5 | Vimentin | 5,6 |
| IPI00448938.1 | H Chain H Igg B12 | 14.7 |
| IPI00697648.1 | Actin alpha 1 skeletales Muskelprotein | 6,5 |
| IPI00022792.3 | hMFAP 4 | 45,1 |
| IPI00455050.1 | sarkomeres Tropomyosin kappa | 87,1 |
| IPI00014581.1 | TPM1 humanes Tropomyosin alpha-Kette | 28,7 |
| IPI00220709.3 | beta Tropomyosin | 52, 4 |
| IPI00010779.3 | Tropomyosin 4 | 20,6 |
| IPI00303568.3 | PTGES 2 | 6, 1 |
| IPI00010796.1 | Prolyl 4-hydroxylase, beta subunit | 4.6 |
| IPI00022391.1 | Amyloid P-Komponente, Serum | 7,3 |
| IPI00216138.5 | Transgelin | 15 |
| IPI00021264.1 | Calponin 1 | 21,1 |
| IPI00022433.5 | Homo sapiens p20 Protein [pir B53814] | 16, 9 |
| IPI00718271.2 | 17 kDa Myosin leichte Kette | 8, 4 |

**Herunterregulierte Proteine:**

| **NCBI-Accession** | **Identifiziertes Protein** | **Fold change** |
|---|---|---|
| IPI00218342.9 | Methylentetrahydrofolatdehydrogenase 1 | - 9,37 |
| IPI00745872.1 | PRO2619 | - 3,2 |
| IPI00218914.4 | Aldehyddehydrogenase 1 | - 7,7 |
| IPI00029717.1 | Fibrinogen alpha-Kette Preprotein | - 6, 4 |
| IPI00218407.5 | Fructose-Bisphosphat-Aldolase B | - 16,6 |
| IPI00020632.4 | Argininosuccinatsynthetase | - 11,0 |
| IPI00014424.1 | Eef1a2 | - 6, 4 |
| IPI00440493.2 | ATP5A1 Protein | - 5, 8 |
| IPI00708487.1 | alpha-2-Actin; alpha cardiac Actin | - 8, 6 |
| IPI00017551.1 | Regucalcin (Seneszenzmarkerprotein 30) | - 13,3 |
| IPI00708398.1 | ABBOS-Serumalbuminprecursor | -13,3 |
| IPI00291006.1 | mitochondrialer Malatdehydrogenaseprecursor | - 5,9 |
| IPI00030363.1 | mitochondrialer Acetoacetyl-CoA-Thiolaseprecursor | - 5,9 |

| | | |
|---|---|---|
| NCBI: National Centre for Biotechnology Information | | |

Im Folgenden werden die Sequenzinformationen zu einigen der identifizierten Proteine angegeben. Dabei sind die Peptidsequenzen dunkel unterlegt, die zum einen zur Identifikation der Proteine führten und zum anderen eine Unterscheidung der Proteinisoformen zulassen (vgl. auch Sequenzprotokoll (sequence listing) als Anhang zur Patentanmeldung).

Tropomyosin

SEQ ID NO 1 MDAIKKKMQMLKLDKENALDRAEQAEADKKAAEDRSKQLEEDIAAKEKLL
SEQ ID NO 2 MDAIKKKMQMLKLDKENALDRAEQAEADKKAAEDRSKQLEEDIAAKEKLL
SEQ ID NO 3 MDAIKKKMQMLKLDKENAIDRAEQAEADKKQAEDRCKQLEEEQQALQKKL
SEQ ID NO 4 MDAIKKKMQMLKLDKENALDRAEQAEADKKAAEDRSKQLEDELVSLQKKL
SEQ ID NO 5 -------------------------------------MAGLNSLEAVKRKI

SEQ ID NO 1 RVSEDERDRVLEELHKAEDSLLAAEEAAAKAEADVASLNRRIQLVEEELD
SEQ ID NO 2 RVSEDERDRVLEELHKAEDSLLAAEEAAAKAEADVASLNRRIQLVEEELD
SEQ ID NO 3 KGTEDEVEKYSESVKEAQEKLEQAEKKATDAEADVASLNRRIQLVEEELD
SEQ ID NO 4 KGTEDELDKYSEALKDAQEKLELAEKKATDAEADVASLNRRIQLVEEELD
SEQ ID NO 5 QALQQQADEAEDRAQGLQRELDGERERREKAEGDVAALNRRIQLFEEELD

SEQ ID NO 1 RAQERLATALQKLEEAEKAADESERGMKVIESRAQKDEEKMEIQEIQLKE
SEQ ID NO 2 RAQERLATALQKLEEAEKAADESERGMKVIESRAQKDEEKMEIQEIQLKE
SEQ ID NO 3 RAQERLATALQKLEEAEKAADESERGMKVIENRAMKDEEKMELQEMQLKE
SEQ ID NO 4 RAQERLATALQKLEEAEKAADESERGMKVIESRAQKDEEKMEIQEIQLKE
SEQ ID NO 5 RAQERLATALQKLEEAEKAADESERGMKVIENRAMKDEEKMEIQEMQLKE

SEQ ID NO 1 AKHIAEDADRKYEEVARKLVIIESDLERAEERAELSEGKCAELEEELKTV
SEQ ID NO 2 AKHIAEDADRKYEEVARKLVIIESDLERAEERAELSEGKCAELEEELKTV
SEQ ID NO 3 AKHIAEDSDRKYEEVARKLVILEGELERSEERAEVAESRARQLEEELRTM
SEQ ID NO 4 AKHIAEDADRKYEEVARKLVIIESDLERAEERAELSEGQVRQLEEQLRIM

SEQ ID NO 5          AKHIAEEADRKYEEVARKLVILEGELERAEERAEVSELKCGDLEEELKNV

SEQ ID NO 1          TNDLKSLEAQAEKYSQKEDRYEEEIKVLSDKLKEAETRAEFAERSVTKLE
SEQ ID NO 2          TNDLKSLEAQAEKYSQKEDRYEEEIKVLSDKLKEAETRAEFAERSVTKLE
SEQ ID NO 3          DQALKSLMASEEEYSTKEDKYEEEIKLLEEKLKEAETRAEFAERSVAKLE
SEQ ID NO 4          DQTLKALMAAEDKYSQKEDRYEEEIKVLSDKLKEAETRAEFAERSVTKLE
SEQ ID NO 5          TNNLKSLEAASEKYSEKEDKYEEEIKLLSDKLKEAETRAEFAERTVAKLE

SEQ ID NO 1          KSIDDLEDELYAQKLKYKAISEELDHALNDMTSI
SEQ ID NO 2          KSIDDLEDELYAQKLKYKAISEELDHALNDMTSI
SEQ ID NO 3          KTIDDLEETLASAKEENVEIHQTLDQTLLELNNL
SEQ ID NO 4          KSIDDLEEKVAHAKEENLSMHQMLDQTLLELNNM
SEQ ID NO 5          KTIDDLEEKLAQAKEENVGLHQTLDQTLNELNCI

Transgelin

SEQ ID NO 6          MANKGPSYGMSREVQSKIEKKYDEELEERLVEWIIVQCGPDVGRPDRGRL
SEQ ID NO 7          MANKGPSYGMSREVQSKIEKKYDEELEERLVEWIIVQRGPDVGRPDRGRL

SEQ ID NO 6          GFQVWLKNGVILSKLVNSLYPDGSKPVKVPENPPSMVFKQMEQVAQFLKA
SEQ ID NO 7          GFQVWLKNGVILSKLVNSLYPDGSKPVKVPENPPSMVFKQMEQVAQFLKA

SEQ ID NO 6          AEDYGVIKTDMFQTVDLFEGKDMAAVQRTLMALGSLAVTKNDGHYRGDPN
SEQ ID NO 7          AEDYGVIKTDMFQTVDLFEGKDMAAVQRTLMALGSLAVTKNDGHYRGDPN

SEQ ID NO 6          WFMKKAQEHKREFTESQLQEGKHVIGLQMGSNRGASQAGMTGYGRPRQII
SEQ ID NO 7          WFMKKAQEHKREFTESQLQEGKHVIGLQMGSNRG----------------

SEQ ID NO 6          S
SEQ ID NO 7          -

SEQ ID NO 1: sarkomeres Tropomyosin kappa, TPM1-kappa; NCBI
Accession: IPI00455050.1

SEQ ID NO 2: sarkomeres Tropomyosin kappa; NCBI Accession:
IPI00455050.1

SEQ ID NO 3: beta Tropomyosin; NCBI Accession: IPI00220709.3

SEQ ID NO 4: TPM 1 humanes Tropomyosin 1 alpha - Kette; NCBI
Accession: IPI00014581.1

SEQ ID NO 5: Tropomyosin 4; NCBI Accession: IPI00010779.3

SEQ ID NO 6: Transgelin; NCBI Accession: IPI00216138.5

SEQ ID NO 7: Transgelinvariante; NCBI Accession: IPI00216138.5

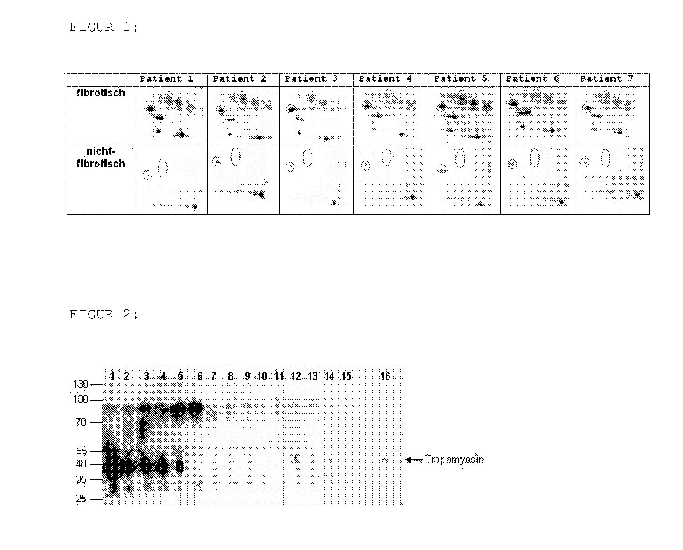

In Figur 1 sind Aufnahmen der mittels 2D-Gelelektrophorese aufgetrennten Proteine gezeigt, wobei fibrotische und nicht-fibrotische Zellen aus zirrhotischem Leberparenchym von insgesamt 7 Patienten verwendet wurden, die unter mit Hepatitis C verbundener Leberzirrhose litten. Die kreisrunde Markierung zeigt Tropomyosin (beta), die ovale Markierung hMFAP4. Die Ähnlichkeit der Aufnahmen unterstreicht die Reproduzierbarkeit der Ergebnisse.

Als besonders erfolgversprechende und bevorzugte erfindungsgemäße Biomarker haben sich Tropomyosin, Transgelin, Calponin, hMFAP4 und Vimentin erwiesen. Bei dem Tropomyosin kann es sich weiterhin um sarkomeres Tropomyosin kappa, beta Tropomyosin, TPM1 humanes Tropomyosin oder Tropomyosin 4 handeln.

Bei dem erwähnten hMFAP4 handelt es sich um das humane mikrofibrilar assoziierte Protein 4. Neben den zuvor erwähnten hochregulierten Proteinen, können jedoch auch herunterregulierte metabolische Enzyme als Marker verwendet werden, insbesondere Argininosuccinatsynthetase, Methylentetrahydrofolatdehydrogenase, Fructose-1,6-Bisphosphataldolase, mitochondriale Malatdehydrogenase und mitochondriale Acetoacetyl-CoA-Thiolase.

Auf einige der identifizierten Proteine wird im folgenden kurz eingegangen, wobei es sich bei den dort angegebenen Erläuterungen lediglich um Erklärungsversuche handelt, die hinsichtlich des Patentbegehrens in keiner Weise beschränkend zu verstehen sind.

Regucalcin, auch als Seneszensmarkerprotein-30 (SMP-30) bekannt, spielt eine bedeutende Rolle bei der Aufrechterhaltung des intrazellulären Ca2+-Niveaus durch Aktivierung von Ca2+-Enzymen in der Plasmamembran, den Mikrosomen und Mitochondrien. Regucalcin wurde 13-fach herabreguliert in fibrotischen im Vergleich zu gesunden Leberzellen. Dies könnte darauf hindeuten, dass die kompensatorischen Effekte von Regucalcin gegenüber oxidativem Stress in erkranktem Lebergewebe vermindert werden.

Mitochondriale Malatdehydrogenase und mitochondriale Acetoacetyltransferase wurden beide um das 5,9-fache im fibrotischen Lebergewebe herunterreguliert. Es konnte gezeigt werden, dass die Wechselwirkung zwischen Hepatitis C-Virus-Coreprotein und den Proteinen NS3 und NS5 mit Mitochondrien zur Ausbildung reaktiver Sauerstoffspezies (ROS) führt, was eine Erklärung für die schwächere Expression der genannten Enzyme in fibrotischen Zellen sein könnte.

Ein weiteres Anzeichen für gestörte mitochondriale Enzyme in fibrotischen Leberzellen ist die verringerte Expression der ATP-Synthase alpha-Untereinheit (ATP5A1), die die ATP-Synthese während der oxidativen Phosphorylierung katalysiert.

Weitere Effekte des Hepatitis C-Virus-Coreproteins und des NS5A-Proteines sind die Assoziierung mit Membranen des endoplasmatischen Reticulums, des Golgi-Apparates und eine Verstärkung der intrazellulären Lipidakkumulation durch Wechselwirkung mit Apolipoprotein A1 (apoA1) oder A2 (apoA2). Das Lipidtransferprotein wird entsprechend inhibiert oder die Synthese von VLDL (very low density lipoproteins) wird gestört. Es konnte eine signifikante Herabregulierung der Acetyl-CoA-Acetyltransferase in fibrotischen Leberzellen beobachtet werden (fold change: -5,88).

Metabolische Störungen in fibrotischer Leber manifestieren sich auch in einer herabgesetzten Expression von glykolitischen Enzymen wie Fructose-1,6-Bisphosphataldolase (fold change: -16,6), Enolase-1 (2-Phosphodiglycerathydrolase) oder Glyoxylase.

Aufgrund dieser metabolischen Disfunktionen ist die Proteinsynthese in fibrotischen Leberzellen teilweise deutlich reduziert: Serumalbumin (fold change: -13,2), welches als Carrier für Fettsäuren, Steroide und Thyroidhormone fungiert und das extrazelluläre Flüssigkeitsvolumen stabilisiert, wurde nur noch vermindert exprimiert.

Der Prozess der Leberregeneration in fibrotischen Leberzellen wird offenbar durch eine schwächere Expression von Methylentetrahydrofolatdehydrogenase beeinträchtigt (fold change: -9,4), welche drei aufeinanderfolgende Reaktionen bei der Umwandlung von C-1-Derivaten von Tetrahydrofolat katalysiert. Eine unverminderte enzymatische Funktion ist für die normale zelluläre Funktion, das Wachstum und die Dedifferenzierung bedeutsam.

Insgesamt scheint eine Störung des zellulären Gleichgewichtes, der glykolytischen Reaktionswege und der Lipidkompartimentierung und des Metabolismus in fibrotischen Zellen vorzuliegen, was die Produktion von reaktiven organischen Spezies (ROS) begünstigt. Diese wiederum induzieren die Synthese von TGF-β1 in Hepatozyten und hepatischen Stelatzellen, den stärksten Promotor von hepatischer Fibrogenese.

Im Bereich der verstärkt nachweisbaren Proteine in fibrotischem Gewebe konnten die Proteine Actin alpha (fold change: 6,5) und Actin gamma (fold change: 9,1) identifiziert werden. Es handelt sich dabei um Hauptkomponenten der dünnen Filamente von Muskelzellen und des Zytoskeletons von Nicht-Muskelzellen. Actin ist offenbar ein Produkt der HSC-Zellen und tritt daher bei Hepatitis C-induzierter Fibrose verstärkt auf.

Ein weiteres identifiziertes Protein ist das Vimentin (fold change: 4,6). Es handelt sich hierbei vermutlich um ein Produkt der hepatischer Stelatzellen aus Mesenchym.

Verschiedene Tropomyosin-Isoformen, die offenbar nicht durch Hepatozyten aber durch myofibroblastartige hepatische Stelatzellen produziert werden, weisen z. T. die höchsten Raten an Verstärkung auf (fold change: 9,7 bis 83,1).

Das 34 kDa Protein Calponin wird normalerweise ebenfalls spezifisch in glatten Muskelzellen exprimiert und bindet Calmodulin, Actin und Tropomyosin. Angesichts der myofibroblastartigen aktivierten hepatischen Stelatzellen wurde gemäß Ergebnis der Proteomanalyse auch Calponin in fibrotischen Zellen heraufreguliert (fold change: 18,5).

Auch Transgelin (fold change: 15) ist vermutlich ein Produkt von hepatischen Stelatzellen. Transgelin ist ein 22 kDa Protein, das auch als SM22-alpha bezeichnet wird, und weist strukturelle Ähnlichkeiten mit Calponin auf. Die Amyloidkomponente P, ein Glykoprotein, das sich aus einem Paar nicht kovalent gebundener Pentamere zusammensetzt, wobei die Untereinheiten eine Größe von 23 bis 25 kDa aufweisen, wurde ebenfalls bei Fibrose stärker exprimiert als in gesundem Gewebe (fold change: 7,3). Die physiologische Funktion in hepatischer Fibrogenese ist bislang unbekannt, aber die Überexpression deutet auf einen abnormalen zellulären Prozess hin.

Zusammengefasst läßt sich sagen, dass die Über- bzw. Unterexpression der als Biomarker verwendbaren Proteine auf ein gestörtes zelluläres Gleichgewicht, beeinträchtigte mitochondriale und metabolische Enzyme, verminderte zelluläre Synthese und eine verstärkte Expression zytoskeletaler Proteine beim Prozess der Apoptose in fibrotischen Leberzellen zurückzuführen ist.

Bei der Untersuchung einer Probe auf die als Biomarker dienenden Proteine kann, wie oben beschrieben, so vorgegangen werden, dass mit Hilfe einer 2D-Gelelektrophorese, bestehend aus isoelektrischer Fokussierung in der ersten Dimension und Gelelektrophorese in der zweiten Dimension eine Auftrennung der Proteine erfolgt und durch Vergleich des Proteinmusters mit einer nicht-fibrotischen Kontrollprobe die Über- bzw. Unterexpression bestimmter Proteine nachgewiesen wird. Bei der Gelelektrophorese handelt es sich bevorzugt um eine SDS-Polyacrylamid-Gelelektrophorese. Entsprechende Software zur Auswertung der Gele ist beispielsweise von GE Healthcare in Form der DeCyder Software erhältlich.

Zum Nachweise der Proteine werden die Proben vor Durchführung der 2D-Gelelektrophorese vorzugsweise mit einem Farbstoff gelabelt. Bei den Farbstoffen handelt es sich vorzugsweise um Fluoreszenzfarbstoffe. Besonders bevorzugt ist der Einsatz von Cy2, Cy3 und/oder Cy5. Diese Farbstoffe sind beispielsweise von der Firma GE Healthcare, Freiburg, Deutschland, erhältlich. Es handelt sich dabei um Carbomethylindocyaninfarbstoffe, wobei zwei Indolmoleküle über eine Kohlenstoffkette mit konjugierten Doppelbindungen verbunden sind. Die entsprechend funktionalisierten Farbstoffe können beispielsweise mit den Thiolgruppen der Seitenketten des Cysteins zur Reaktion gebracht werden, um die Proteine mit den Farbstoffen kovalent zu verknüpfen. Zwecks Reduktion von Disulfidbrücken wird dabei die Probe zunächst mit Hilfe eines geeigneten Reduktionsmittels reduziert, beispielsweise mit Hilfe von Tris(2-Carboxyethyl)phosphinhydrochlorid (TCEP). Anschließend wird mit entsprechend funktionalisiertem Farbstoff umgesetzt, bis schließlich die Reaktion durch Zugabe von DTT gestoppt wird. Denkbar ist jedoch auch die Funktionalisierung anderer Aminosäurereste mit Hilfe der Farbstoffe, beispielsweise der Seitenkette des Lysins.

Die Verwendung des Cy3, Cy5-Farbstoffsystems ist insofern besonders vorteilhaft als neben der eigentlichen Probe auch ein interner Standard bei der 2D-Gelelektrophorese eingesetzt werden kann, wobei die eigentliche Probe und der interne Standard mit unterschiedlichen Farbstoffen (Cy3 bzw. Cy5) versehen werden.

Selbstverständlich sind neben den genannten Farbstoffen auch andere (Fluoreszenz)farbstoffe einsetzbar, die aus dem Stand der Technik sind, beispielsweise Fluorescein oder Tetramethylrhodamin.

Der Nachweis und die Quantifizierung der als Marker dienenden Proteine kann auch mit Hilfe weiterer, dem Fachmann geläufiger Protein-Diagnoseverfahren durchgeführt werden, insbesondere unter Verwendung radioaktiv oder fluoreszenzmarkierter Antikörper. Zu nennen sind hier insbesondere dazu geeignete bioanalytische Verfahren, wie zum Beispiel Immunhistochemie, Antikörperarrays, Luminex, ELISA, Immunfluoreszenz, Radioimmunoassays. Der Nachweis und die Quantifizierung der als Marker dienenden Proteine kann auch mit weiteren dazu geeigneten bioanalytischen Verfahren, wie zum Beispiel massenspektrometrischen Verfahren, z.B. MRM (Multi reaction monitoring) oder AQUA (absolute Quantification), mit deren Hilfe die Markerproteine quantitativ gemessen werden können, durchgeführt werden.

Bei der zum Nachweis der Proteine verwendeten Probe kann es sich um eine Probe von Lebergewebe handeln, die mit Hilfe einer Biopsie entnommen wurde. Möglich ist jedoch auch die Verwendung von (Voll-)Blut-, Serum- oder Plasmaproben, welche selbstverständlich leichter zu gewinnen sind.

Neben den beschriebenen Verfahren betrifft die Erfindung auch die Verwendung der genannten Proteine als Biomarker zum Nachweis einer Leberentzündung insbesondere einer hepatischen Fibrose.

Die genannten erfindungsgemäßen Markerproteine können in weiteren Ausführungsformen ebenfalls zur Differentialdiagnose, insbesondere differentialdiagnostischen Früherkennung, Verlaufsprognose der Lebererkrankung, Beurteilung des Schweregrades, therapiebegleitenden Verlaufsbeurteilung, Ätiologie und in-vitro Diagnostik eingesetzt werden.

In einer weiteren Ausführungsform betrifft die Erfindung einen Kit oder diagnostische Vorrichtung zur Durchführung des erfindungsgemäßen Verfahrens, wobei der Kit mindestens einen erfindungsgemäßen Biomarker (auch: Markerprotein) samt Nachweisreagenzien und weitere Hilfsmittel enthält.

Nachfolgende Beispiele dienen zur Erläuterung der Erfindung, ohne die Erfindung auf diese Beispiele zu beschränken.

### Beispiel

Beispiel 1 - Probenanalyse:
Von insgesamt sieben Patienten mit einer Hepatitis C-Infektion, Genotyp 1, die sich einer Lebertransplantation unterzogen, wurde Leberparenchym entnommen, sofort auf Eis gekühlt und bei -86°C gelagert. Unter dem Mikroskop wurden Gewebeproben aufgetrennt zwischen fibrotischem Gewebe und gesunden Abschnitten. Das fibrotische Material wurde dabei entlang des fibrotischen Septums entnommen. Die Schichten für die 2D-Gelelektrophorese wurden mit Haematoxylin angefärbt und bei -20°C gelagert. Die isolierten Zellen aus der Mikrodissektion wurden in 100 µl Lysispuffer (Tris HCl 30 mM; Thioharnstoff 2 M; Harnstoff 7 M, CHAPS 4%; pH 8,0) aufgenommen und anschließend durch Anwendung von Ultraschall aufgebrochen (6 x 10 s Pulse).
Mit einer Zentrifugation (12.000 g für 5 min) wurden DNA und sonstige Zellreste entfernt. Die Proteinkonzentration des Lysats wurde ermittelt.
Zur Anfertigung eines internen Standards wurden 3 µg des Gewebelysates durch Anwendung von 2 nmol Tris-(2-Carboxyethyl)-phosphinhydrochlorid (TCEP) bei 37°C im Dunkeln über eine Zeitspanne von einer Stunde reduziert. Cy-Farbstoffe (GE Healthcare, Freiburg, Deutschland) wurden mit wasserfreiem DMF p. a. (2 nmol/µl) verdünnt und 4 nmol Cy3 wurden den mit Hilfe von TCEP reduzierten Proben zugegeben. Nach einer Inkubationszeit von 30 min bei 37°C wurde die Labeling-Reaktion durch Zugabe von 4 µl DTT (1,08 g/ml) gestoppt.
Die Cystein-Aminosäuren der zu untersuchenden Proteine (3.500 fibrotische Zellen, 2.500 nicht-fibrotische Zellen) wurden ebenfalls durch Inkubation mit 2 nmol TCEP bei 37°C im Dunkeln für eine Stunde reduziert, bevor 4 nmol Cy5 hinzugegeben wurden. Nach gründlicher Durchmischung wurden die Proben 30 min lang bei 37°C im Dunkeln umgesetzt. Die Reaktion wurde gestoppt durch Zugabe von 10 µl DTT.
Zur Vorbereitung der isoelektrischen Fokussierung wurden 10 µl Ampholine 2-4 (GE Healthcare) hinzugefügt. Die Cy3- und Cy5- markierten Zellen wurden nach Durchmischung gleichzeitig weiter verarbeitet.
Anschließend wurde eine 2D-Gelelektrophorese durchgeführt, wobei zur isoelektrischen Fokussierung ein 21,25 h Spannungsgradient verwendet wurde. Als Äquilibrierungspuffer wurde 125 mM Tris, 40 % (w/v) Glycerol, 3 % (w/v) SDS, 65 mM DTT, pH 6,8 für 10 min verwendet. Anschließend wurde in der zweiten Dimension eine Polyacrylamidgelektrophorese durchgeführt.
Mit Hilfe eines geeigneten Scanners (Typhoon 9400, GE Healthcare) wurden nach beendeter Gelelektrophorese Bilder aufgenommen und mit Hilfe der ImageQuant-Software und der DeCyder-Software (GE Healthcare) ausgewertet. Auf diese Weise konnte eine differenzielle Analyse im Gel (DIA) durchgeführt werden, um die einzelnen Spots zu detektieren und zu quantifizieren.
Mit Hilfe des Enzyms Trypsin in 10 mM Ammoniumbicarbonatpuffer (pH 7,8) wurden die Proteine bei 37°C über Nacht gespalten. Die auf diese Weise generierten Fragmente wurden zweifach mit einem Acetonitril-Ameisensäuregemisch extrahiert zur weiteren Auftrennung und massenspektrometrischen Untersuchung. Dies erfolgte mit Hilfe von Online-RP-Kapillar-HPLC, gekoppelt mit nano-ESI-MS (Elektrosprayionisations-Massenspektrometrie). Mit Hilfe der HPLC-MS-Kopplung und unter Heranziehung geeigneter Proteindatenbanken (NCBI, National Centre for Biotechnology Information) konnte eine vollständige Identifizierung der Proteinspots herbeigeführt werden.

Beispiel 2: Protein Tropomyosin und seine Detektion in Patientenseren:
Hierfür wurde eine Proteindetektion mittels Western Blot-Analyse durchgeführt. Das Tropomyosin-Protein konnte in Patientenseren von Leberzirrhose- Patienten unterschiedlicher Genese reproduzierbar identifiziert werden (Figur 2).
Figur 2: Tropomyosin Western Blot von folgenden Patientenseren:
   - 1:: (Patient 1) Hepatitis C (HepC) Zirrhose CHILD C
   - 2:: (Patient 2) HepC Zirrhose CHILD C
   - 3:: (Patient 3) HepC Zirrhose CHILD C
   - 4:: (Patient 4) HepC Zirrhose CHILD C
   - 5:: (Patient 5) HepC Zirrhose CHILD C
   - 6:: (Patient 6) HepC Zirrhose CHILD A
   - 7:: (Patient 7) HepC Zirrhose CHILD A
   - 8:: (Patient 8) Normalkontrolle
   - 9:: (Patient 9) Normalkontrolle
   - 10:: (Patient 10) Normalkontrolle

   - 11:: (Patient 11) Normalkontrolle
   - 12:: (Patient 12) Äthyltoxische Zirrhose CHILD C
   - 13:: (Patient 13) Äthyltoxische Zirrhose CHILD C
   - 14:: (Patient 14) HepB Fibrose
   - 15:: (Patient 15) HepC Fibrose
   - 16:: Tropomyosin 0,005µg
Bei den Patienten handelte es sich zum einen um Hepatitis C infizierte Patienten mit Leberzirrhose bzw. Fibrose, zum anderen um Patienten mit äthyltoxischer Leberzirrhose sowie einem Hepatitis B Patienten mit Leberfibrose. Patienten mit Leberzirrhose wurden weiter in verschiedene CHILD-Klassen unterteilt, welche Aufschluss über den Schweregrad der Zirrhose, durch Einbezug verschiedener Blutparameter, geben. Die Klassifizierung erfolgt von CHILD A mit einer Überlebensrate von ca. 100% für das nächste Jahr bis CHILD C mit einer Überlebensrate von ca. 30%. Eine Tropomyosinbande konnte in Patientenseren gesunder Normalkontrollen nicht detektiert werden (Figur 2, Bande 8-11). Interessanterweise konnte Tropomyosin in Hepatitis C Patienten mit einer CHILD C Klassifizierung nachgewiesen werden (Figur 2, Bande 1-5), während bei Zirrhotikern mit einer CHILD A Zirrhose keine Proteinbande detektierbar war (Figur 2, Bande 6-7). Zusätzlich wurden äthyltoxische Leberzirrhosen untersucht (CHILD C Klassifizierung), die ebenfalls ein Signal für Tropomyosin in abgeschwächter Form zeigten und eine CHILD C Klassifizierung aufwiesen (Figur 2, Bande 12-13). Weiterhin konnte im Hepatitis B Fibrosepatienten eine Tropomyosinbande detektiert werden, was im Hepatitis C Fibrosepatienten nicht möglich war.

## Patentansprüche

1. In-vitro Verfahren zur Untersuchung biologischer Proben eines Menschen auf Leberentzündung insbesondere hepatische Fibrose und / oder Leberzirrhose,
wobei die Probe auf eines oder mehrere Proteine als Marker für Leberentzündung, insbesondere hepatische Fibrose und / oder Leberzirrhose, untersucht wird, wobei eine gegenüber dem gesunden Zustand erhöhte Konzentration der Proteine das Vorliegen einer Leberentzündung, insbesondere einer hepatischen Fibrose und / oder Leberzirrhose, anzeigt,
**dadurch gekennzeichnet,**
**dass** die Proteine ausgewählt sind aus der Gruppe bestehend aus: Tropomyosin, Vimentin, Actin alpha 1 skeletales Muskelprotein, hMFAP 4, Transgelin, Calponin 1 oder jeweils eine Teilsequenz davon.

2. Verfahren nach Anspruch 1, wobei die Proteine eine Kombination aus Tropomyosin und hMFAP 4 oder Tropomyosin und hMFAP 4 und Calponin 1 oder / und Transgelin ist.

3. Verfahren nach Anspruch 1 oder 2, **dadurch gekennzeichnet, dass** das Tropomyosin: sarkomeres Tropomyosin kappa, beta Tropomyosin, TPM 1 humanes Tropomyosin oder Tropomyosin 4 oder eine Kombination davon ist.

4. Verfahren zur Diagnose von Leberentzündungen, insbesondere hepatische Fibrose und / oder Leberzirrhose,
**dadurch gekennzeichnet,**
**dass** eine Bestimmung mindestens eines Proteins ausgewählt aus der Gruppe bestehend aus: Tropomyosin, Vimentin, Actin alpha 1 skeletales Muskelprotein, hMFAP 4, Transgelin, Calponin 1 oder jeweils eine Teilsequenz davon oder jeweils eine Teilsequenz davon von einem zu untersuchenden Patienten durchgeführt wird.

5. Verfahren nach einem der Ansprüche 1 bis 3, **dadurch gekennzeichnet, dass** die Untersuchung der Probe mit Hilfe eines Diagnostikums durchgeführt wird, das dazu geeignete bioanalytische Verfahren, wie zum Beispiel Immunhistochemie, Antikörperarrays, Luminex, ELISA, Immunfluoreszenz, Radioimmunoassays verwendet.

6. Verfahren nach einem der Ansprüche 1 bis 4, **dadurch gekennzeichnet, dass** die Untersuchung der Probe mittels massenspektrometrischer Verfahren wie MRM (Multi reaction monitoring) oder AQUA (absolute Quantification) erfolgt, mit deren Hilfe die Markerproteine quantitativ bestimmbar sind.

7. Verfahren nach einem der Ansprüche 1 bis 5, **dadurch gekennzeichnet, dass** die Untersuchung der Probe mit Hilfe einer 2D-Elektrophorese erfolgt, wobei in der ersten Dimension eine isoelektrische Fokussierung, in der zweiten Dimension eine Gelelektrophorese durchgeführt wird.

8. Verfahren nach Anspruch 6, **dadurch gekennzeichnet, dass** die Gelelektrophorese eine SDS-Polyacrylamid-Gelelektrophorese ist.

9. Verfahren nach Anspruch 6 oder 7, **dadurch gekennzeichnet, dass** die Proben vor Durchführung der 2D-Gelelektrophorese mit einem Farbstoff gelabelt werden.

10. Verfahren nach Anspruch 8, **dadurch gekennzeichnet, dass** der Farbstoff Cy2, Cy3 und/oder Cy5 ist.

11. Verfahren nach einem der Ansprüche 1 bis 9, **dadurch gekennzeichnet, dass** die Probe eine Leberbiopsieprobe ist.

12. Verfahren nach einem der Ansprüche 1 bis 10, **dadurch gekennzeichnet, dass** die Probe aus Blutserum, Blutplasma oder (Voll)Blut erhalten wird.

13. Verwendung von Tropomyosin, Vimentin, Actin alpha 1 skeletales Muskelprotein, hMFAP 4, Transgelin, Calponin 1 als Marker zum Nachweis einer Leberentzündung, insbesondere einer hepatischen Fibrose und / oder Leberzirrhose.

14. Verwendung mindestens eines der in Anspruch 12 genannten Markerproteine zur Differentialdiagnose, insbesondere differentialdiagnostischer Früherkennung, Verlaufsprognose der Lebererkrankung, Beurteilung des Schweregrades, therapiebegleitende Verlaufsbeurteilung, Ätiologie und in-vitro Diagnostik.

15. Kit oder diagnostische Vorrichtung enthaltend mindestens ein Markerprotein nach Anspruch 12 samt Nachweisreagenzien und weitere Hilfsmittel.
